# EUROPÄISCHE PATENTSCHRIFT

(11) **EP 1 354 905 B1**
(45) Veröffentlichungstag und Bekanntmachung des Hinweises auf die Patenterteilung: **11.01.2006**
(21) Anmeldenummer: 03007647.5
(22) Anmeldetag: 03.04.2003
(51) Int. Cl.: C07C 51/367, C08G 65/332

(54) **Verwendung von Ethercarbonsäuren mit niedrigem Stockpunkt**
Use of ether-carboxylic acids having low pour point
Utilisation des acides ether-carboxyliques à bas point d'écoulement

(30) Priorität: 18.04.2002 DE 10217208
(43) Veröffentlichungstag der Anmeldung: 22.10.2003
(73) Patentinhaber: Clariant GmbH, 65929 Frankfurt am Main (DE)
(72) Erfinder: Dahlmann, Uwe, Dr., 69126 Heidelberg (DE); Kupfer, Rainer, Dr., 65795 Hattersheim (DE); Masvidal Climent, Eduard, 43001 Tarragona (ES)
(74) Vertreter: Mikulecky, Klaus

(56) Entgegenhaltungen:
- EP-A- 0 344 442
- EP-A- 0 566 956
- EP-A- 0 897 906
- WO-A-99/23281
- DE-C- 19 928 128
- US-A- 5 233 087

## Beschreibung

Die vorliegende Erfindung betrifft die Verwendung von Ethercarbonsäuren mit niedrigem Stockpunkt, sowie deren Verwendung als Emulgator mit antikorrosiven Eigenschaften in Metallbearbeitungshilfsmittel, in Kosmetikformulierungen und als Detergenzien in Waschmitteln.

Ethercarbonsäuren, d.h. organische Carbonsäuren, die neben der Carboxylfunktion eine oder mehrere Etherbrücken tragen, bzw. deren Alkali- oder Aminsalze, sind als milde Detergenzien mit hohem Kalkseifendispergiervermögen bekannt. Sie finden sowohl in Waschmittel- und Kosmetikformulierungen, aber auch in technischen Anwendungen, wie z.B. Metallbearbeitungsflüssigkeiten und Kühlschmiermittel, Verwendung. Ethercarbonsäuren (ECS) werden gemäß dem Stand der Technik entweder durch Alkylierung von Alkohol- oder Fettalkoholoxethylaten oder -oxpropylaten mit Chloressigsäurederivaten (Williamsonsche Ethersynthese) oder aus den gleichen Ausgangsprodukten durch Oxidation mit verschiedenen Reagenzien (Luftsauerstoff, Hypochlorit, Chlorit) unter Katalyse mit verschiedenen Katalysatoren hergestellt. Die Williamsonsche Ethersynthese stellt vor allem aufgrund der Kosten- Wirkung-Beziehung das technisch geläufigste Verfahren für die Herstellung von ECS dar, jedoch besitzen durch dieses Verfahren hergestellte Produkte noch gravierende Mängel in Bezug auf die Handhabbarkeit für den Anwender, wie beispielsweise Löslichkeitsverhalten, Aggregatzustand bei niedrigen Temperaturen und Lagerstabilität.

Diese Mängel sind im wesentlich auf verfahrensbedingte Nebenbestandteile zurückzuführen. So werden trotz Verwendung von Überschüssen des entsprechenden Chloressigsäurederivats nur Umsätze von ca. 70-85 % erreicht, so dass Restmengen an Oxethylat und Fettalkohol, der dem Oxethylat zugrunde liegt, im Endprodukt verbleiben. Des weiteren entstehen durch den zu verwendenden Überschuss des Chloressigsäurederivats Folgeprodukte, wie beispielsweise Glykolsäure, Diglycolsäure und deren Derivate, die eine wesentliche Ursache für die Alterung der Produkte sind und gegebenenfalls Probleme beim Löslichkeitsverhalten hervorrufen können.

Ein weiterer Nachteil der Williamson- Synthese besteht in der hohen Belastung der Reaktionsprodukte durch Natriumchlorid (Gehalt ca. 1 %), das in wässrigen Lösungen eine wesentliche Ursache für Lochfraß- Korrosion darstellt.

DE-A-199 28 128 offenbart ein Verfahren zur Herstellung von Ethercarbonsäuren mit niedrigem Restalkoholgehalt, indem Fettalkohole zunächst unter Einsatz nichtkatalytischer Mengen an Alkalikatalysator (NaOH, KOH, Alkoholate über 5 Mol-%) mit Alkylenoxiden umgesetzt werden, und die resultierenden, hoch alkalischen Reaktionsmischungen, die aus einem Gemisch von oxethylierten Alkoholen und Alkoholaten verschiedener Polyalkylenglykolether bestehen, anschließend in einer klassischen Williamson-Synthese mit Natriumchloracetat in die entsprechende Ethercarbonsäure überführt werden. Durch dieses Verfahren wird zwar der Restgehalt an Fettalkohol in der Ethercarbonsäure ohne spezielle Katalysatoren verringert, jedoch kann die Bildung der oben beschriebenen Nebenprodukte nicht vermieden werden.

EP-A-0 897 906 offenbart ein Verfahren zur Herstellung von Ethercarbonsäuren durch Reaktion einer Polyoxyalkylenverbindung mit einer Halogenessig- oder -propionsäure in einem organischen Lösemittel, und einer nachfolgenden Aufreinigung des erhaltenen Produkts, so dass es für pharmazeutische Anwendungen geeignet ist.

WO-99/23281 offenbart, dass Ethercarbonsäuren als Bestandteile korrosionsinhibierender Metallbearbeitungsflüssigkeiten geeignet sind.

EP-A-0 566 956 beschreibt Korrosionsschutzmittel die 0-30 Gew.-% Ethercarbonsäuren der Formel R³-O-(CH₂CH₂)O)ₙ-CH₂-COOH enthalten, wobei R³ gleich C₁₀-C₂₂-Alkyl, C₁₀-C₂₂-Alkenyl oder Alkaryl ist und n gleich 1 bis 10.

Es bestand daher die Aufgabe, ein Verfahren zur Herstellung von Ethercarbonsäuren zu entwickeln, durch das der Gehalt an ungewünschten Nebenprodukten, wie Natriumchlorid und Glykolsäure reduziert werden kann.

Überraschenderweise wurde gefunden, dass die nach einen Waschprozess mit Sulfatlösung erhaltenen Ethercarbonsäuren nicht nur einen geringeren Anteil an Nebenprodukten aufweisen, sondern vor allem auch einen niedrigeren Stockpunkt als auf herkömmlichem Wege hergestellte Ethercarbonsäuren aufweisen. Des weiteren zeigten die Untersuchungen, dass diese Ethercarbonsäuren auch einen unerwartet niedrigen Elektrolyt- Gehalt aufweisen, der direkt durch Leitfähigkeitsmessungen verifizierbar ist, und der das Stockpunktsverhalten eindeutig bedingt.
Gegenstand der Erfindung ist daher die Verwendung von Verbindungen der Formel (1) 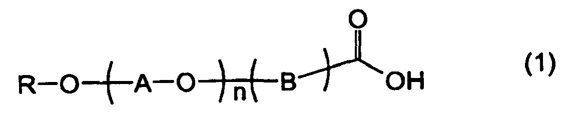 oder deren Salze der Formel (2) 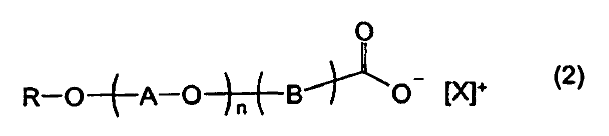 worin
- A: Cr bis C₄-Alkylen,
- B: C₁- bis C₄-Alkylen
- n: eine Zahl von 1 bis 100, und
- R: C₁- bis C₃₀-Alkyl, C₂- bis C₃₀-Alkenyl, oder C₆- bis C₃₀-Aryl
- X: ein Kation

bedeuten, welche hergestellt werden,
indem man ein basisches Gemisch von oxethylierten Alkoholen der Formel 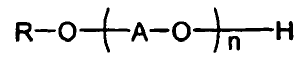 und deren Alkoholaten mit einer C₂- bis C₅-Chlorcarbonsäure alkyliert und das so erhaltene basische Zwischenprodukt nach Absäuerung durch Waschen mit wässriger Sulfatlösung so lange aufreinigt, bis die so erhaltene Ethercarbonsäure eine Leitfähigkeit von < 1000 µS/cm besitzt, als Emulgator mit antikorrosiven Eigenschaften.

Ein weiterer Gegenstand der Erfindung ist die Verwendung von Schwefelsäure zur Absäuerung des erhaltenen basischen Intermediats und somit die Erzeugung der zum Waschen benötigten Sulfatlösung in situ.

A bedeutet vorzugsweise Propylen oder Ethylen, insbesondere Ethylen. In einer weiteren bevorzugten Ausführungsform der Erfindung steht die Gruppe -(A-O)ₙ- für eine gemischte Alkoxygruppe, die Ethylen-, Propylen- und Butylenreste enthalten kann. Handelt es sich um eine gemischte Alkoxygruppe, so liegt das Verhältnis der vom Ethylenoxid abgeleiteten Gruppen zu den von Propylen- oder Butylenoxid abgeleiteten Gruppen vorzugsweise zwischen 10:1 und 1:1. n steht vorzugsweise für eine Zahl zwischen 2 und 70, insbesondere 3 bis 50.

B steht vorzugsweise für eine geradkettige Alkylengruppe, insbesondere für Methylen. B kann auch für eine verzweigte Alkylengruppe mit 3 oder 4 Kohlenstoffatomen stehen.

R bedeutet in einer bevorzugten Ausführungsform einen C₈-C₂₄, insbesondere einen C₁₂-C₁₈-Alkyl- oder Alkenylrest. Steht R für einen aromatischen Rest, so ist ein Phenylrest mit Alkylsubstitution zwischen 4 und 12 Kohlenstoffatomen bevorzugt.

X kann in einer bevorzugten Ausführungsform für Wasserstoffionen stehen. In einer weiteren bevorzugten Ausführungsform steht X für Alkali- oder Erdalkaliionen, insbesondere Lithium, Natrium, Kalium, Magnesium, oder Calcium.

In einer weiteren bevorzugten Ausführungsform werden als Kationen Ammoniumionen der Formel NR¹R²R³R⁴ verwendet, wobei R¹, R², R³ und R⁴ unabhängig voneinander H, C₁- bis C₂₂-Alkyl, C₆- bis C₁₈-Aryl, C₇- bis C₂₂-Alkylaryl und/oder C₁- bis C₂₂-Alkenyl bedeuten können. Die Reste R¹, R², R³ und R⁴ können Heteroatome wie N, P, O, S enthalten. Die Ammoniumreste können Monoalkylammonium-, Dialkylammonium-, Trialkylammonium- oder Tetraalkylammoniumreste sein, worin die Alkylsubstituenten unabhängig voneinander mit bis zu 3 Hydroxygruppen besetzt sein können. Vorzugsweise steht X für Ammoniumreste, die einen, zwei, drei oder vier C₂- bis C₁₀-Alkylreste tragen. In einer weiteren bevorzugten Ausführungsform können einer, zwei oder drei der Reste R¹ bis R⁴ alkoxyliert sein.

Geeignete Amine für die Herstellung von Ammoniumkationen X sind Monoamine mit primärer oder sekundärer Aminofunktion wie Methylamin, Ethylamin, Butylamin, Laurylamin, Cocosfettamin, Stearylamin, Dimethylamin, Diethylamin, Dibutylamin, aber auch Di- und Polyamine wie z.B. 3-Dimethylaminopropylamin, 3-Diethylaminopropylamin, 3-Morpholinopropylamin, Diethylentriamin, Triethylentetramin oder Tetraethylenpentamin.

Geeignete Aminoalkohole für die Herstellung von Ammoniumkationen X sind beispielsweise N,N-Dimethylaminoethanol, N,N-Diethylaminoethanol, N,N-Dibutylaminoethanol, 3-Dimethylaminopropanol, N-Hydroxyethylmorpholin, Monoethanolamin, Diethanolamin, Triethanolamin, 3-Aminopropanol, Isopropanolamin, 2-(2-Aminoethoxy)ethanol und Cyclohexylamino-N,N-diethanol.

Als Basisfettalkohol für das hier beschriebene Verfahren eignen sich lineare oder verzweigte, gesättigte oder ungesättigte Fettalkohole mit 1-30 C-Atomen sowie Alkylphenole mit einem C₁-C₂₀ Alkylrest, bevorzugt sind C₆-C₂₂ Fettalkohole.

Diese können gemäß des Standes der Technik mit Alkylenoxiden, z.B. Ethylenoxid, Propylenoxid, Butylenoxid oder Mischungen verschiedener solcher Alkylenoxide umgesetzt werden, wobei Ethylenoxid oder Mischungen aus Ethylenoxid und Propylenoxid bevorzugt sind. Bezogen auf Fettalkohol werden 1-30 mol Alkylenoxid beaufschlagt, bevorzugt 1-12 mol. Die Reaktionstemperaturen liegen hierbei bei ca. 80-160°C.

Im anschließenden Reaktionsschritt wird die Alkoholat/Alkoholoxethylat-Mischung mit einem Chlorcarbonsäurederivat und einer Base, bevorzugt trockenem Chloressigsäure-Natriumsalz und Natriumhydroxid umgesetzt. Dies kann geschehen, indem man die Oxethylat/Alkoholat-Mischung mit 100-150 Mol-% Natriumchloracetat bei 30-100°C umsetzt und gleichzeitig oder nacheinander mit festem Natriumhydroxid oder Kaliumhydroxid versetzt, so dass die Summe aus der in der Oxethylat-/Alkoholatmischung vorliegenden Base und der zusätzlich zugegebenen Basenmenge der Menge an Natriumchloracetat entspricht.

Nach der Alkylierungsreaktion kann die intermediär erzeugte Lösung des Ethercarbonsäure-Alkalisalzes durch Absäuerung auf pH < 3 mit einer beliebigen Säure auf pH < 3 abgesäuert werden. Die so erhaltene freie Ethercarbonsäure wird nach Abtrennung der wässrigen Phase mehrmals durch Waschen mit einer Sulfatlösung aufgereinigt.

Die Absäuerung wird bevorzugt mit Schwefelsäure durchgeführt, da auf diese Weise bereits Alkalisulfatlösung in situ erzeugt wird, mit der bereits der erste Waschschritt durchgeführt werden kann. Die Absäuerung kann auch mit Salzsäure erfolgen.

Als Sulfatlösung wird bevorzugt gesättigte Natriumsulfatlösung verwendet.

Isolierung und Waschprozess der Ethercarbonsäure erfolgt durch gleichmäßige Durchmischung und anschließende Phasentrennung oberhalb des Trübungspunktes.

Wie die folgenden Beispiele zeigen, können nach dem hier offenbarten Verfahren Ethercarbonsäuren mit niedrigem Stockpunkt und geringem Elektrolyt-Gehalt hergestellt werden.

### Beispiele

### A) Herstellverfahren

### Beispiel 1 (Vergleich) (Oleylalkohol + 10 EO - ECS, Absäuerung mit H₂SO₄):

In einer 21 Rührapparatur wurden 412 g (0,565 mol) Oleylalkohol + 10 EO (z.B. Genapol O 100) unter Stickstoffspülung vorgelegt und auf 40°C erwärmt. Dann wurden unter gutem Rühren 92,0 g (0,79 mol) Chloressigsäure-Natriumsalz eingetragen und die Reaktionsmischung auf 50 °C erwärmt. Anschließend wurden insgesamt 35,0 g (0,88 mol) Natriumhydroxid-Microprills portionsweise so zugegeben, dass die Innentemperatur 55°C nicht übersteigt. Nach jeder Zugabe wurde jeweils 30 min, nach der letzten Zugabe 2 h bei 70°C nachgerührt. Die Reaktionsmischung wurde dann auf 90°C erwärmt und dann warme Schwefelsäure (15 - 20 %ig) zulaufen lassen, bis ein pH-Wert < 3 erreicht ist. Die Reaktionsmischung wurde dann gleichmäßig durchmischt, auf ca. 100°C erhitzt und in ein beheizbares Trenngefäß mit Rührung und Bodenventil überführt. Phasentrennung erfolgte ohne Rühren bei einer Temperatur von ca. 100-110°C, wobei nach einer Trennzeit von ca. 5 h 552 g wässrige Unterphase und 448 g Produkt als hell gelbe Flüssigkeit erhalten wurden.

### Beispiel 2 (Oleylalkohol + 10 EO - ECS, Absäuerung mit H₂SO₄ und Waschen mit Natriumsulfat-Lösung):

Herstellung der Oleylalkohol + 10 EO - ECS erfolgte nach Beispiel 1. Nach Abtrennung der wässrigen Unterphase wurden 90 g einer 25 - 28 %igen Natriumsulfat-Lösung in Wasser zugegeben und die Mischung 30 min bei ca. 100°C kräftig durchmischt. Phasentrennung erfolgte nach 2 h wiederum ohne Rühren bei einer Temperatur von ca. 100-110°C, wobei die Waschphase durch das Bodenventil abgelassen wurde. Die Waschung wird wenigstens 3 mal wiederholt. Es wurden 427 g Produkt als hell gelbe Flüssigkeit erhalten.

### Beispiel 3 (Oleylalkohol + 10 EO - ECS, Absäuerung mit HCl und Waschen mit Natriumsulfat-Lösung):

In einer 2 l Rührapparatur wurden 412 g (0,565 mol) Oleylalkohol + 10 EO (z.B. Genapol O 100) unter Stickstoffspülung vorgelegt und auf 40°C erwärmt. Dann wurden unter gutem Rühren 92,0 g (0,79 mol) Chloressigsäure-Natriumsalz eingetragen und die Reaktionsmischung auf 50°C erwärmt. Anschließend wurden insgesamt 35,0 g (0,88 mol) Natriumhydroxid-Microprills portionsweise so zugegeben, dass die Innentemperatur 55°C nicht übersteigt. Nach jeder Zugabe wurde jeweils 30 min, nach der letzten Zugabe 2 h bei 70°C nachgerührt. Die Reaktionsmischung wurde dann auf 90°C erwärmt und dann warme Salzsäure (35 %ig) zu zulaufen lassen bis ein pH-Wert < 3 erreicht ist. Die Reaktionsmischung wurde dann gleichmäßig durchmischt, auf ca. 100°C erhitzt und in ein beheizbares Trenngefäß mit Rührung und Bodenventil überführt. Phasentrennung erfolgte nach einer Trennzeit von ca. 5 h ohne Rührung bei einer Temperatur von ca. 100-110°C. Nach Abtrennung der wässrigen Unterphase wurden 90 g einer 25-28 %igen Natriumsulfat-Lösung in Wasser zugegeben und die Mischung 30 min bei ca. 100°C kräftig durchmischt. Phasentrennung erfolgte nach 2 h wiederum ohne Rühren bei einer Temperatur von ca. 100-110°C, wobei die Waschphase durch das Bodenventil abgelassen wurde. Die Waschung wird wenigstens 3 mal wiederholt. Nach dem letzten Waschschritt wurden 440 g Produkt als hell gelbe Flüssigkeit erhalten.

**Tabelle 1: Kennzahlen der Ethercarbonsäuren (SZ = Säurezahl)**

| Beispiel | Gehalt NaCl [%] | Gehalt Sulfat [%] | Leitfähigkeit [µS/cm] | SZ [mg KOH/g] | SZ nach Lagerung [mg KOH/g] | Stockpunkt [°C] |
|---|---|---|---|---|---|---|
| Vergleich | 0,64 | 0 | 1503 | 70,2 | 61,1 | 28 |
| 1 (Vergleich) | 0,36 | 0,63 | 2216 | 74,9 | 74,6 | 22 |
| 2 | 0,046 | 0,036 | 751 | 76,6 | 76,0 | 12 |
| 3 | 0,081 | 0,042 | 805 | 67,7 | 67,5 | 12 |

Als Vergleich wurde die kommerziell erhältliche Ethercarbonsäure Emulsogen® COL 100 herangezogen. Es handelt sich dabei im wesentlichen um eine Ethercarbonsäure der Zusammensetzung Oleyl-O-(EO)₁₀-CH₂-COOH, die nach einem Verfahren des Standes der Technik hergestellt wurde.

Wie aus der Tabelle 1 ersichtlich ist, zeichnen sich die durch das hier offenbarte Verfahren hergestellten Ethercarbonsäuren durch geringe Elektrolytbelastung aus, die sich durch eine geringe Leitfähigkeit manifestiert. Des weiteren führt der geringe Gehalt an Elektrolyten zu dem sekundären Effekt, dass die Ethercarbonsäuren ein signifikant verändertes Stockpunktverhalten aufweisen, was die Anwendung der Produkte bei niedrigen Temperaturen beim Verbraucher vereinfacht.
Ein weiterer Vorteil der durch dieses Verfahren hergestellten Ethercarbonsäuren ergibt sich aus der längeren Lagerstabilität, die durch die unveränderten Säurezahlen nach Lagerung dokumentiert wird.

### B) Verwendung der erfindungsgemäßen Verbindungen als Korrosionsinhibitor für wassermischbare Kühlschmiermittel, Reinigungsflüssigkeiten, und für Oberflächenbehandlungen.

Die Korrosionsschutzprüfung erfolgte in Anlehnung an die DIN-Norm 51360, Teil 2 (Filterpapiertest) und dient der Beurteilung der Korrosion von Eisenmetall. Als Maß für die Korrosion dienen Art und Anzahl der Korrosionsabzeichnungen auf einem Rundfilter, die durch Einwirken eines mit Wasser gemischten Kühlschmierstoffes (KSS) auf genormte Graugussspäne (Spangröße: 3 bis 6 mm²) entstehen. Die Beurteilung erfolgt durch Sichtprüfung und Einstufung des Korrosionsgrades (1 bis 4) nach Vergleichstabelle.

Als Vergleich wurden ebenfalls die kommerziell erhältliche Ethercarbonsäure Emulsogen® COL 100 herangezogen.

Die zu prüfenden Produkte wurden für die Untersuchungen zum Korrosionsschutz mit Triethanolamin (TEA) unter Bildung des entsprechenden Ammoniumsalzes auf pH 9,0 eingestellt.

**Tabelle 2: Korrosionsschutzprüfung nach DIN (Filterpapiertest), Angabe in Korrosionsgraden 1 bis 4 nach Vergleichstabelle DIN-Norm 51360, Teil 2 (Filterpapiertest), Konzentrationen in Gew.-%**

| Beispiel | ECS | Konzentration der ECS | | |
|---|---|---|---|---|
| | | 3% | 4% | 5% |
| 4 | Vergleich | 4 | 2 | 1 |
| 5 | 1 (Vergleich) | 4 | 2 | 1 |
| 6 | 2 | 4 | 1 | 0-1 |
| 7 | 3 | 4 | 1 | 1 |

Wie aus der Tabelle 2 ersichtlich ist, führt der geringe Gehalt an Elektrolyten nicht nur zu niedrigen Stockpunkten, sondern auch zu verbessertem Korrosionsschutzverhalten der erfindungsgemäß verwendeten Ethercarbonsäuren.

## Patentansprüche

1. Verwendung von Verbindungen der Formel (1) 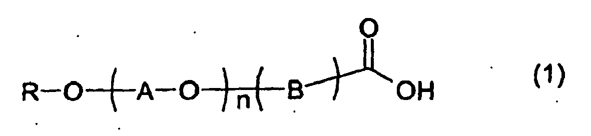 oder deren Salze der Formel (2) 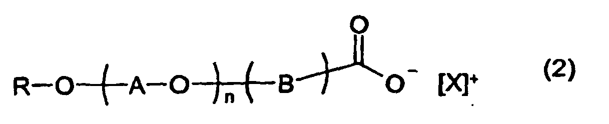 worin
A C₂- bis C₄-Alkylen,
B C₁- bis C₄-Alkylen
n eine Zahl von 1 bis 100,
R C₁- bis C₃₀-Alkyl, C₂- bis C₃₀-Alkenyl, oder C₆- bis C₃₀-Aryl, und
X ein Kation
bedeuten,
welche hergestellt werden indem man ein basisches Gemisch von oxethylierten Alkoholen der Formel 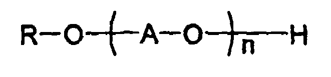 und deren Alkoholaten mit einer C₂- bis C₅-Chlorcarbonsäure alkyliert und das so erhaltene basische Zwischenprodukt nach Absäuerung durch Waschen mit wäßriger Sulfatlösung so lange aufreinigt, bis die so erhaltene Ethercarbonsäure eine Leitfähigkeit von < 1000 µS/cm besitzt, als Emulgator mit antikorrosiven Eigenschaften.

2. Verwendung gemäß Anspruch 1, worin A für Propylen oder Ethylen steht.

3. Verwendung gemäß Anspruch 1 und/oder 2, worin n für eine Zahl zwischen 2 und 70 steht.

4. Verwendung gemäß einem oder mehreren der Ansprüche 1 bis 3, worin B für eine Methylengruppe steht.

5. Verwendung gemäß einem oder mehreren der Ansprüche 1 bis 4, worin R für einen C₈- bis C₂₄-Alkyl- oder Alkenylrest steht.

6. Verwendung gemäß einem oder mehreren der Ansprüche 1 bis 5, worin Schwefelsäure zur Absäuerung verwendet wird.

7. Verwendung gemäß einem oder mehreren der Ansprüche 1 bis 6 in Metallbearbeitungsmitteln, Kosmetikformulierungen oder Waschmitteln.

## Claims

1. The use of compounds of the formula (1) 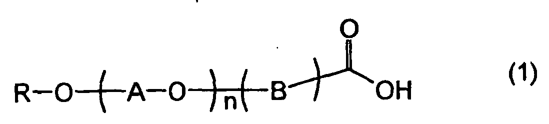 or salts thereof of the formula (2) 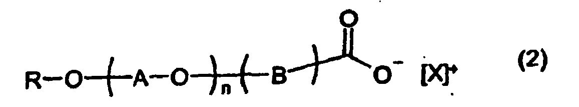 in which
A is C₂- to C₄-alkylene,
B is C₁- to C₄-alkylene
n is a number from 1 to 100, and
R is C₁- to C₃₀-alkyl, C₂- to C₃₀-alkenyl, or C₆- to C₃₀-aryl, and
X is a cation
which are prepared by alkylating a basic mixture of oxethylated alcohols of the formula 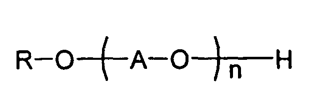 and alkoxides thereof with a C₂- to C₅-chlorocarboxylic acid, and purifying the basic intermediate obtained in this way, following acidification, by washing with aqueous sulfate solution until the ether carboxylic acid obtained in this way has a conductivity of < 1000 µS/cm, as emulsifier with anticorrosive properties.

2. The use as claimed in claim 1, in which A is propylene or ethylene.

3. The use as claimed in claim 1 and/or 2, in which n is a number between 2 and 70.

4. The use as claimed in one or more of claims 1 to 3, in which B is a methylene group.

5. The use as claimed in one or more of claims 1 to 4, in which R is a C₈- to C₂₄-alkyl or alkenyl radical.

6. The use as claimed in one or more of claims 1 to 5, in which sulfuric acid is used for the acidification.

7. The use as claimed in one or more of claims 1 to 6 in metal-working compositions, cosmetic formulations or washing compositions.

## Revendications

1. Utilisation de composés de formule (1) 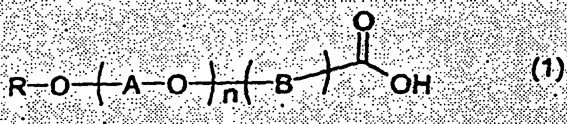 ou de leurs sels de formule (2) 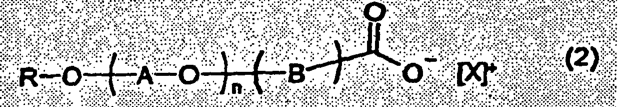 dans lesquelles
A représente un alkylène en C₂ à C₄,
B représente un alkylène en C₁ à C₄,
n représente un nombre de 1 à 100,
R représente un alkyle en C₁ à C₃₀, alcényle en C₂ à C₃₀ ou aryle en C₆ à C₃₀, et
X représente un cation,
qui sont préparés en alkylant un mélange basique d'alcools éthoxylés de formule 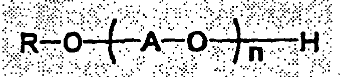 et de leurs alcoolates avec un acide chlorocarboxylique en C₂ à C₅ et en purifiant le produit intermédiaire basique ainsi obtenu après acidification par lavage avec une solution aqueuse de sulfate jusqu'à ce que l'acide éther-carboxylique ainsi obtenu possède une conductivité de < 1 000 µS/cm, comme émulsifiant avec des caractéristiques anticorrosives.

2. Utilisation selon la revendication 1, dans laquelle A représente le propylène ou l'éthylène.

3. Utilisation selon la revendication 1 et/ou 2, dans laquelle n représente un nombre entre 2 et 70.

4. Utilisation selon une ou plusieurs des revendications 1 à 3, dans laquelle B représente un groupe méthylène.

5. Utilisation selon une ou plusieurs des revendications 1 à 4, dans laquelle R représente un radical alkyle ou alcényle en C₈ à C₂₄.

6. Utilisation selon une ou plusieurs des revendications 1 à 5, dans laquelle on utilise de l'acide sulfurique pour l'acidification.

7. Utilisation selon une ou plusieurs des revendications 1 à 6 dans les agents d'usinage des métaux, les formulations de cosmétiques ou les détergents.
